(19) Europäisches Patentamt
European Patent Office
Office européen des brevets

(11) Publication number: **0 353 944 B1**

# (12) EUROPEAN PATENT SPECIFICATION

(45) Date of publication of patent specification: **11.10.95**

(51) Int. Cl.[6]: **C07D 213/82**

(21) Application number: **89307631.5**

(22) Date of filing: **27.07.89**

The file contains technical information submitted after the application was filed and not included in this specification

## (54) Aminocarbonyl-substituted pyridinesulfinic acid or salts thereof.

(30) Priority: **04.08.88 JP 194812/88**
**12.08.88 JP 201525/88**
**23.08.88 JP 208770/88**
**17.04.89 JP 97219/89**

(43) Date of publication of application:
**07.02.90 Bulletin 90/06**

(45) Publication of the grant of the patent:
**11.10.95 Bulletin 95/41**

(84) Designated Contracting States:
**CH DE FR GB IT LI NL**

(56) References cited:
**EP-A- 0 232 067**

(73) Proprietor: **ISHIHARA SANGYO KAISHA, LTD.**
**No. 3-22, Edobori 1-chome**
**Nishi-ku**
**Osaka (JP)**

(72) Inventor: **Haga, Takahiro Ishihara Sangyo K.K.**
**Chuo Kenkyusho, 3-1**
**Nishishibukawa 2-chome**
**Kusatsu-shi**
**Shiga-ken (JP)**
Inventor: **Tsujii, Yasuhiro Ishihara Sangyo K.K.**
**Chuo Kenkyusho, 3-1**
**Nishishibukawa 2-chome**
**Kusatsu-shi**
**Shiga-ken (JP)**
Inventor: **Isogai, Tatsuo Ishihara Sangyo K.K.**
**Yokkaichi Plant**
**1, Ishihara-cho**
**Yokkaichi-shi**
**Mie-ken (JP)**
Inventor: **Awazu, Takao Ishihara Sangyo K.K.**
**Chuo Kenkyusho, 3-1**
**Nishishibukawa 2-chome**
**Kusatsu-shi**
**Shiga-ken (JP)**
Inventor: **Murai, Shigeo Ishihara Sangyo K.K.**
**Chuo Kenkyusho, 3-1**
**Nishishibukawa 2-chome**
**Kusatsu-shi**
**Shiga-ken (JP)**
Inventor: **Tanaka, Toshihiro Ishihara Sangyo K.K.**
**Chuo Kenkyusho, 3-1**
**Nishishibukawa 2-chome**
**Kusatsu-shi**
**Shiga-ken (JP)**

EP 0 353 944 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

(74) Representative: **Pearce, Anthony Richmond et al**
**MARKS & CLERK,**
**Alpha Tower,**
**Suffolk Street Queensway**
**Birmingham B1 1TT (GB)**

## Description

The present invention relates to novel aminocarbonyl-substituted pyridinesulfinic acid (hereinafter referred to as ACPS), or salts thereof as a precursor of aminosulfonyl-substituted pyridinecarboxylic acid amide compound (hereinafter referred to as APCA) thereof useful as the starting material of agricultural chemicals, medicines, and the like, and to an industrially advantageous process for preparing the same.

European Patent Application Laid-Open Nos. 237,292 and 232,067 disclose a process for preparing APCA. However, the process disclosed therein is not always satisfactory to be industrially carried out because of low yield, increased reaction steps, necessity of isolation in respective reaction steps, or of using reactants which are difficult to handle and expensive.

The present inventors made various studies in order to find out an industrially advantageous process for preparing APCA, and, as the result, found out that a process by way of ACPS or salts thereof according to the present invention is capable of providing the intended effects to complete the present invention.

(Disclosure of the Invention)

The present invention provides an aminocarbonyl-substituted pyridinesulfinic acid having the general formula (I):

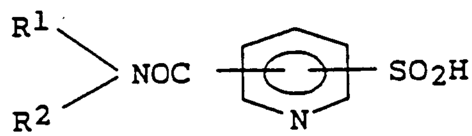

... (I)

where $R^1$ and $R^2$ are a hydrogen atom or a $C_1$-$C_6$ alkyl group respectively, or a salt thereof.

Examples of $C_1$-$C_6$ alkyl group for $R^1$ and $R^2$ of the general formula (I) include for example, methyl, ethyl, propyl and butyl. Examples of the salts include salts of alkali metals or alkaline earth metals such as lithium, sodium, potassium, magnesium, calcium; amine salts due to amines substituted with hydrocarbon group such as triethylamine, dimethylamine; quaternary ammonium salts such as ammonium salt due to ammonia.

Of the above ACPS or salts thereof, a compound having the general formula (I"):

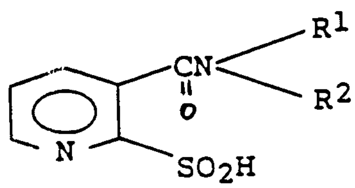

... (I")

wherein $R^1$ and $R^2$ are as defined above, or salts thereof are preferred, and such a combination that $R^2$ is methyl is more preferred, whilst such a combination that $R^1$ is a hydrogen atom or a methyl group and $R^2$ is a methyl group in the general formula (I") is even more preferred. Of the above ACPS or salts thereof, the salts are preferred. Of the salts, alkali metal salts and ammonium salts are preferred, and sodium salt is more preferred.

The above ACPS or salts thereof according to the present invention may be prepared by the following process:

[A]

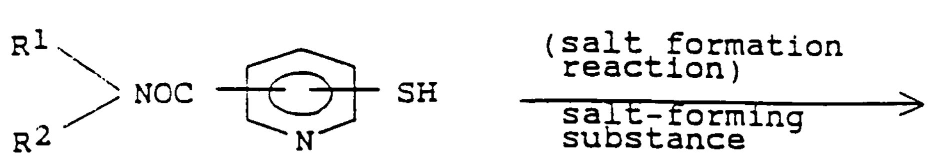

Salt of

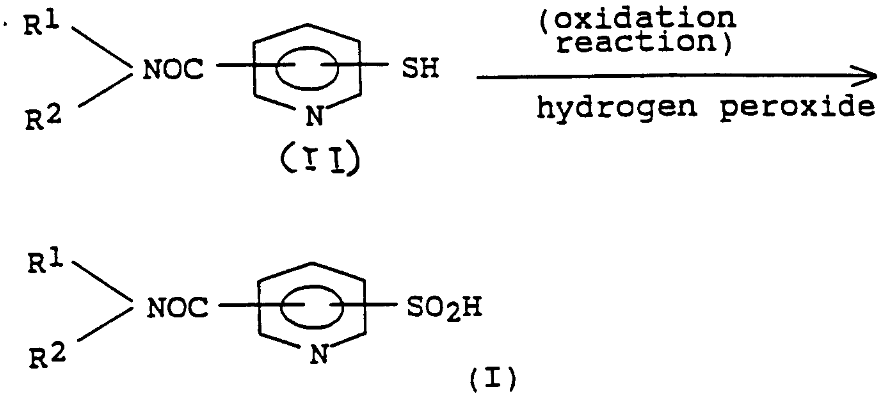


(II)

Salt of

(I)

where $R^1$ and $R^2$ are as defined above. Preferably, the mercapto group and the sulfinic acid group in (II) and (I) above, respectively, are located at the 2-position on the pyridine ring whilst the carboxylic acid amide group is located at the 3-position on the pyridine ring.

The salt formation reaction and the oxidation reaction are explained in detail below.

(Salt Formation Reaction)

The salt formation reaction is normally carried out by reacting a mercapto-substituted pyridinecarboxylic acid amide compound having the general formula (II) (hereinafter referred to as MPCA) with a salt-forming substance in the presence of a solvent such as water. The amount of the solvent used is 100 to 1000% by weight relative to the weight of MPCA. Examples of the salt-forming substance include hydroxides or carbonates of alkali metal or alkaline earth metal such as lithium, sodium, potassium, magnesium, calcium; amine such as triethylamine, dimethylamine; ammonia such as aqueous ammonia solution, ammonia gas; sodium hydroxide and ammonia being preferred. The amount of the salt-forming substance used is a reacting equivalent or more relative to MPCA, for example, 1 to 2 moles when the alkali metal salts are used, or 4 to 20 moles when ammonia is used, per one mole of MPCA respectively.

The reaction temperature may not be generally defined, but is normally 0 to 50 °C, and the reaction time is generally 5 to 60 minutes.

The reaction product may be subjected to the conventional purification and separation procedures to obtain the intended salts of MPCA, or may be used for the following oxidation reaction as it is.

(Oxidation Reaction)

The oxidation reaction is carried out by dissolving or suspending the salt of MPCA in a solvent such as water followed by dropping thereinto hydrogen peroxide, or is carried out by dropping hydrogen peroxide into the reaction product obtained from the above salt formation reaction. The concentration of hydrogen peroxide is not specifically limited, but may be normally about 30% by weight, and its amount to be used is normally 1.8 to 3 moles per one mole of the salt of MPCA. The reaction temperature is not to be generally defined, but is normally 0 to 100 °C, and the reaction time is generally 10 to 60 minutes.

The reaction product may be subjected to the conventional purification and separation procedures as in the case of the above salt formation reaction to isolate the salt of ACPS, neutralization of which makes it possible to obtain ACPS.

The ACPS or salts thereof, which are obtained by the above reaction, may easily be lead to APCA by amination reaction or by oxidation•condensation reaction as described below.

In an industrial practice, the salt of ACPS, which is obtained by the above reaction, may be subjected to amination reaction or to oxidation•condensation reaction as described below, in situ without being isolated. In the above case, the salt of ACPS, which is applicable to oxidation•condensation reaction, is limited to ammonium salt, when a salt other than ammonium salt of ACPS is formed, it is once converted into ammonium salt by salt conversion reaction. The salt conversion reaction is explained below.

(Salt Conversion Reaction)

The salt conversion reaction is carried out by dissolving or suspending alkali metal salts, alkaline earth metal salts or amine salts of ACPS in a solvent such as water followed by generally adding ammonia and subsequently an acid, or is carried out by generally adding ammonia and subsequently an acid to the reaction product containing the alkali metal salt, alkaline earth metal salt, or amine salt of ACPS, which has been obtained according to the above oxidation reaction. Specific examples of the ammonia used include an aqueous ammonia solution, ammonia gas. The amount of the ammonia used is normally 4 to 20 moles per one mole of the alkali metal salt, alkaline earth metal salt or amine salt of ACPS. Examples of the acid include inorganic acid or organic acid such as sulfuric acid, hydrochloric acid, nitric acid, phosphoric acid, acetic acid, sulfuric acid being preferred. The amount of the acid used is 1 to 4 moles per one mole of the alkali metal salt, alkaline earth metal salt or amine salt of the above ACPS. The reaction temperature is not generally defined, but is normally 0 to 50 ° C, and the reaction time is generally 10 to 60 minutes.

The reaction product may be subjected to the conventional purification and separation procedures to isolate ammonium salt of ACPS, but may be applicable to oxidation•concentration reaction, which is explained below, as it is in the same manner as in the case of the above reaction.

The ACPS or alkali metal salts thereof in the present invention may also be prepared according to the following alternate process.

[B]

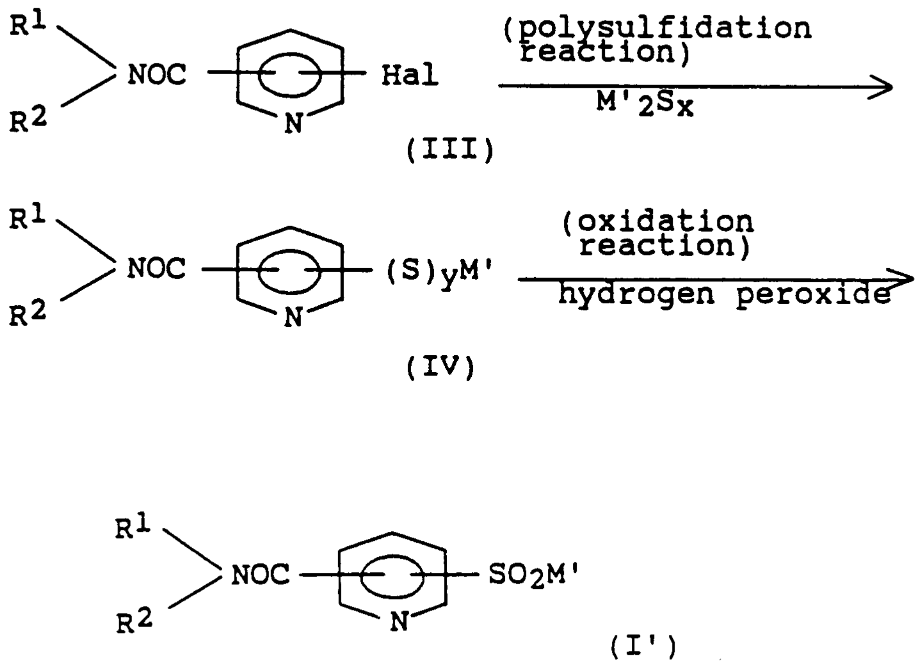


(III)

(IV)

(I')

wherein $R^1$ and $R^2$ are as defined above, M' is an alkali metal element (preferably sodium), x is 2 to 8, and y is 1 to 8. Preferably, the $-SO_2M'$ group is located at the 2-position and the $-CONR_1R_2$ group is located at the 3-position, respectively, on the pyridine ring.

The above polysulfidation reaction and oxidation reaction will be explained in detail.

(Polysulfidation Reaction)

On polysulfidation reaction, polysulfide used may be obtained by reacting one mole of a mixture of hydroxide and hydrosulfide of alkali metal, or of sulfide of alkali metal with 1 to 7 moles, preferably 1 to 2 moles of sulfur beforehand according to the conventional process, or may be obtained by reacting the above reactants in the presence of aminocarbonyl-substituted halogenopyridine compound (hereinafter referred to as ACHP) having the general formula (III) to be prepared in the reaction system and to be directly used in situ. Examples of the alkali metal for the hydroxide, hydrosulfide or sulfide of alkali metal include lithium, sodium, potassium, sodium being preferred. The amount of the hydroxide, hydrosulfide or

sulfide to be used is respectively 0.75 to 5 moles, preferably 1 to 1.5 moles per one mole of ACHP. In this reaction, normally water is used as a solvent, but organic solvent may also be used so long as it is miscible with polysulfide and water. Examples of the organic solvent include lower alcohol such as methanol, ethanol, propanol; polyalcohol such as ethylene glycol, propylene glycol; ethers such as tetrahydrofuran; an aprotic polar solvent such as dioxane, dimethylsulfoxide; ketones such as methyl ethyl ketone; nitriles such as acetonitrile. The amount of the solvent used is normally 10 to 1000% by weight, preferably 10 to 100% by weight relative to the weight of ACHP. Other reaction conditions in this reaction may not generally be defined, but the reaction temperature is normally 0 °C to a reflux temperature, preferably 80 to 150 °C, the reaction pressure is atmospheric pressure to several atms., and the reaction time is generally 0.5 to 30 hours.

The alkali metal salt of pyridinecarboxylic acid amide (poly)sulfide having the general formula (IV), which is obtained in the above reaction is applicable to the following oxidation reaction as it is.

(Oxidation Reaction)

The oxidation reaction may be carried out in the same manner as in the oxidation reaction of the above process [A].

The ACPS or alkali metal salts thereof, which are obtained according to this reaction, may easily be lead to APCA by an amination reaction explained below or the above salt conversion reaction - an oxidation•condensation reaction explained below.

Typical examples of ACPS or salts thereof in the present invention, which are obtained according to the above processes [A] and [B], are shown in Table 1 as follows.

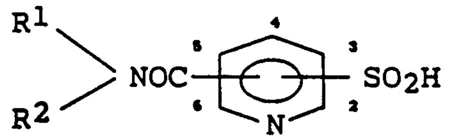

... (I)

or salts thereof.

Table 1

| Compound No. | General formula (I) or salts thereof: Substituted position of $R^1R^2NOC-$ | $R^1$ | $R^2$ | Substituted position of $-SO_2H$ | Kinds of Salts | Physical Properties-melting point (°C) |
|---|---|---|---|---|---|---|
| 1 | 3 | $CH_3$ | $CH_3$ | 2 | -- | -- |
| 2 | " | " | " | " | Na salt | 260 - 265 (decomposed) |
| 3 | " | " | " | " | $NH_4$ salt | 128 - 130 |
| 4 | " | H | " | " | Na salt | -- |
| 5 | " | " | $C_3H_7$ (iso) | " | " | -- |
| 6 | " | $C_2H_5$ | $C_2H_5$ | " | triethyl amine salt | -- |
| 7 | " | $CH_3$ | $CH_3$ | 4 | Ca salt | -- |
| 8 | " | " | " | 2 | K salt | -- |

Next, the MPCA having the general formula (II) and used as a starting material in the above process [A] may be prepared according to the following process.

[a]

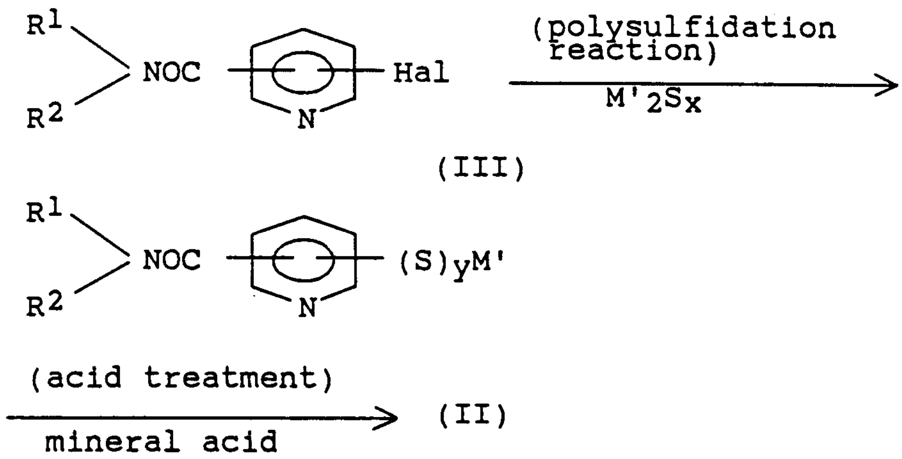


(III)

(II)

wherein R[1], R[2], Hal, M', x and y are as defined above.

The above polysulfidation reaction and acid treatment are explained more in detail.

(Polysulfidation Reaction)

The polysulfication reaction may be carried out in the same manner as in the polysulfidation reaction of the above process [B].

(Acid Treatment)

The conventional acid treatment of a reaction product obtained according to the above polysulfidation reaction and containing an alkali salt of pyridinecarboxylic acid amide (poly)sulfide having the general formula (IV) results in liberating the intended MPCA, evolving hydrogen sulfide gas, and in generating sulfur. The acid treatment is carried out by adding a mineral acid having no oxidative action, for example, concentrated hydrochloric acid or dilute sulfuric acid, to the reaction product so that pH may show 3 or less, followed by conventional purification and separation procedures to isolate the intended MPCA.

Further, the ACPS or salts thereof in the present invention may be lead to aminosulfonyl-substituted pyridinecarboxylic acid amide compound (APCA) having the following general formula (V) by the following amination reaction or oxidation•condensation reaction.

[b-1]

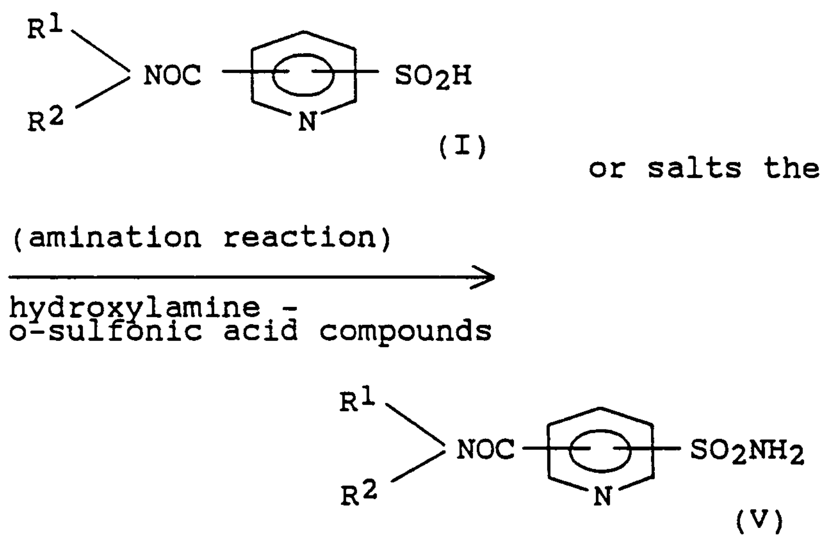


(I)

or salts thereof

(V)

where R[1] and R[2] are as defined above.

(Amination Reaction)

The amination reaction is carried out by dissolving or suspending the above ACPS or salts thereof in a solvent such as water and adding thereto hydroxylamine-o-sulfonic acid compounds, or by adding hydroxylamine-o-sulfonic acid compounds to the reaction product obtained according to the above oxidation reaction for reacting. Preferably, a basic substance is further added in the above reaction. The order of addition of the hydroxylamine-o-sulfonic acid compounds and of the basic substance is not specifically limited, but it is preferred to first add the hydroxylamine-o-sulfonic acid compounds and then add the basic substance. Examples of hydroxylamine-o-sulfonic acid compounds include hydroxylamine-o-sulfonic acid, o-mesitylene sulfonylhydroxylamine, hydroxylamine-o-sulfonic acid being preferred. The amount of the hydroxylamine-o-sulfonic acid compounds used is 1 to 3 moles per one mole of ACPS or salts thereof. Examples of the basic substance include sodium acetate, sodium carbonate, potassium carbonate, sodium hydrogencarbonate, sodium phosphate, disodium hydrogenphosphate, sodium hydroxide, potassium hydroxide, ammonia, triethylamine, ammonia, sodium acetate and sodium hydroxide being preferred. The amount of the basic substance used is 0.5 to 4 reacting equivalents per one reacting equivalent of ACPS or salts thereof. The reaction temperature is not generally defined, but is normally 0 to 100 °C, and the reaction time is generally 10 minutes to 24 hours.

The reaction product may be subjected to the conventional purification and separation procedures to obtain APCA.

[b-2]

$NH_4$ salt of

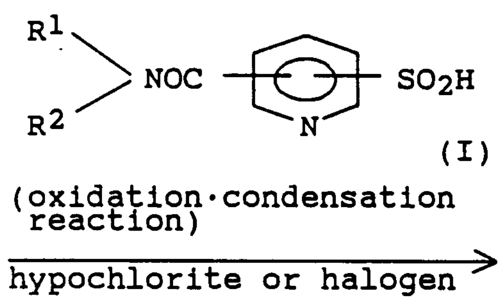


(I)

(V)

where $R^1$ and $R^2$ are as defined above.

(Oxidation•Condensation Reaction)

The oxidation•condensation reaction is carried out by dissolving or suspending ammonium salt of ACPS in a solvent such as water and adding thereto hypochlorite or halogen, or by adding hypochlorite or halogen to the reaction product obtained according to the above oxidation or salt conversion reaction and containing ammonium salt of ACPS for reacting. Examples of hypochlorite include sodium hypochlorite, potassium hypochlorite, calcium hypochlorite, and examples of halogen include bromine, chlorine, iodine, sodium hypochlorite being preferred. The amount to be used of the hypochlorite or halogen is 1 to 4 moles per one mole of ammonium salt of ACPS. The reaction temperature is not generally defined, but is normally -10 to +50 °C, and the reaction time is generally 10 to 60 minutes.

The reaction product may be subjected to neutralization followed by the conventional purification and separation procedures to obtain APCA.

In the above process [b-2], addition of an acid makes it possible to increase yield. The acid may be added at any time during a time period from the MPCA ammonium salt formation reaction step or the reaction step of salt conversion to ammonium salt of ACPS to the oxidation•condensation reaction step, for example, between after the MPCA ammonium salt formation reaction and before the oxidation reaction, between after the oxidation reaction of ammonium salt of MPCA and before the oxidation•condensation reaction. Examples of the acid include inorganic acid or organic acid such as sulfuric acid, hydrochloric acid, nitric acid, phosphoric acid, acetic acid, sulfuric acid being preferred. The amount of the acid used is normally 1 to 5 moles per one mole of respective reaction products in the MPCA ammonium salt formation reaction, the salt conversion reaction to ammonium salt of ACPS, the oxidation reaction, or in the oxidation•condensation reaction.

The APCA obtained according to the above reaction may easily be lead to a substituted pyridinesulfonamide compound such as N-[(4,6-dimethoxypyrimidine-2-yl)aminocarbonyl]-3-dimethylaminocarbonyl-2-pyridinesulfonamide and salts thereof, which are useful as herbicide for use in the corn field as disclosed, for example, in European Patent Application Laid-Open No. 232,067.

(Example)

Examples, which are not to give any limitations to the process of the present invention, are shown below in order to describe more in detail the process of the present invention.

Example 1

Preparation of the starting material, MPCA:

(Polysulfidation Reaction and Acid Treatment)

A mixture of 55.4 g of 2-chloro-N,N-dimethylnicotinamide, 24 g of 70% purity sodium hydrosulfide, 9.6 g of sulfur, 12 g of sodium hydroxide and 15 mℓ of water is refluxed for about 2 hours by heating with agitation to form sodium salt of N,N-dimethylnicotinamide-2-polysulfide. To the above product are added 150 mℓ of water and 30 mℓ of a 50% aqueous sulfuric acid solution under stirring for 30 minutes at 60 to 70 °C, and the generated sulfur is filtered off with warming. The sulfur is washed with 100 mℓ of warm water, and the filtrate and the washing liquor are combined together to obtain a solution containing 2-mercapto-N,N-dimethylnicotinamide. The solution is cooled down to 10 °C, and deposited crystals are collected by filtration. Further, the filtrate is thickened to such an extent that the volume is reduced to about 1/3, and the deposited crystals are collected by filtration. These crystals are combined and dried to obtain 46.5 g of 2-mercapto-N,N-dimethylnicotinamide having a melting point of 200 to 208 °C.

Preparation of ACPS Salt of the Present Invention:

(Salt Formation Reaction)

A 300 mℓ four-necked flask equipped with thermometer, dropping funnel and stirrer is charged with 18.2 g (0.1 mole) of 2-mercapto-N,N-dimethylnicotinamide, 4.4 g (0.11 mole) of sodium hydroxide and 50 mℓ of water to be dissolved with agitation for being reacted at room temperature for 10 minutes to obtain a reaction product containing sodium-N,N-dimethylnicotinamide-2-thiolate.

Separately, the same reaction as above is carried out to obtain a reaction product. The reaction product is thickened, and the deposited crystals are filtered off and dried to isolate 20.0 g of sodium-N,N-dimethylnicotinamide-2-thiolate having a melting point of 260 to 268 °C (a little decomposed).

(Oxidation Reaction)

Into the reaction product obtained according to the above salt formation reaction is dropped with agitation for about 30 minutes 22.7 g (0.2 mole) of 30% aqueous hydrogen peroxide solution at a temperature of 10 to 20 °C externally cooling for reacting to obtain a reaction product containing sodium N,N-dimethylnicotinamide-2-sulfinate.

Separately, the salt formation reaction and oxidation reaction is carried out in the same manner as above to obtain a reaction product. The reaction product is thickened and dried to isolate 22.0 g of sodium N,N-dimethylnicotinamide-2-sulfinate (yield based on MPCA: 93.2%) having a melting point of 260 to 265 °C (colored brown, decomposed).

(Salt Conversion Reaction)

To the reaction product obtained according to the above oxidation reaction as it is, is added 60.7 g (1.0 mole) of 28% ammonia water, and 12.3 g (0.125 mole) of concentrated sulfuric acid at a temperature of 10 to 20 °C is then dropped thereinto with agitation for 15 minutes for reacting to obtain a reaction product containing ammonium salt of N,N-dimethylnicotinamide-2-sulfinic acid.

Separately, the salt formation reaction, oxidation reaction and the salt conversion reaction are carried out in the same manner as above to obtain a reaction product. The reaction product is thickened and dried,

and the residue is extracted with methanol and the extract liquor is dried to isolate 20.9 g of ammonium salt of N,N-dimethylnicotinamide-2-sulfinic acid (yield based on MPCA: 90.5%) having a melting point of 128 to 130 ° C.

Referential Example 1

Preparation of APCA:

(Oxidation • Condensation Reaction)

Into the reaction product obtained according to the salt conversion reaction in Example 1, as it is, is dropped with agitation for about 30 minutes 32 g (0.2 mole) of bromine at a temperature of 10 to 20 ° C for reacting, followed by stirring for 30 minutes and neutralizing with concentrated sulfuric acid to pH 3-6 to deposit white crystals. This reaction product is cooled down to about 20 ° C, and is then filtered, washed with water and dried to obtain 16.2 g (yield based on MPCA: 70.7%; purity: 96.5%) of 2-aminosulfonyl-N,N-dimethylnicotinamide.

Example 2

Preparation of ACPS Salt of the Present Invention:

(Salt Formation Reaction)

A 300 mℓ four-necked flask equipped with thermometer, dropping funnel and stirrer is charged with 9.1 g (0.05 mole) of 2-mercapto-N,N-dimethylnicotinamide and 45.5 g of 28% ammonia water, followed by dissolving with agitation and by reacting for 10 minutes at room temperature to obtain a reaction product containing ammonium-N,N-dimethylnicotinamide-2-thiolate.

The ammonium-N,N-dimethylnicotinamide-2-thiolate (9.7 g) has a melting point of 198 to 201 ° C (partly decomposed).

(Oxidation Reaction)

Into the reaction product obtained according to the above salt formation reaction, as it is, is dropped with agitation for about 15 minutes 11.4 g (0.10 mole) of 30% aqueous hydrogen peroxide solution at a temperature of 5 to 20 ° C externally cooling for reacting, followed by dropping thereinto with agitation for about 20 minutes 12.5 g (0.125 mole) of concentrated sulfuric acid at a temperature of 5 to 20 ° C to obtain a reaction product containing ammonium salt of N,N-dimethylnicotinamide-2-sulfinic acid.

Separately, the ammonium salt formation reaction and oxidation reaction is carried out in the same manner as above to obtain a reaction product. The reaction product is thickened and dried, and the residue is extracted with methanol, followed by drying the extract liquor to isolate 10.8 g (yield based on MPCA: 93.5%) of ammonium salt of N,N-dimethylnicotinamide-2-sulfinic acid having a melting point of 128 to 130 ° C.

Referential Example 2

Preparation of APCA:

(Oxidation • Condensation Reaction)

Into the reaction product obtained according to the oxidation the oxidation reaction in Example 2, as it is, is dropped with agitation for about 30 minutes 62 g (0.1 mole) of 12% sodium hypochlorite solution at a temperature of 5 to 20 ° C for reacting, followed by neutralizing with concentrated sulfuric acid to pH 3-6 to deposit white crystals. This reaction product is cooled down to about 20 ° C, followed by filtering, washing with water and by drying to obtain 8.5 g (yield based on MPCA: 74.2%; purity: 95.6%) of 2-aminosulfonyl-N,N-dimethylnicotinamide.

Example 3

Procedures of Example 2 are repeated except that dropping of 12.5 g (0.125 mole) of concentrated sulfuric acid after the completion of the oxidation reaction is replaced by dropping of 24.5 g (0.125 mole) of 50% sulfuric acid to obtain 10.5 g (yield based on MPCA: 90.9%) of ammonium salt of N,N-dimethylnicotinamide-2-sulfinic acid.

Referential Example 3

Procedures of Referential Example 2 are repeated except that the reaction product obtained according to the oxidation reaction in Example 3 is used as it is and 62 g of 12% sodium hypochlorite solution is replaced by 28 g (0.175 mole) of bromine to obtain 6.98 g (yield based on MPCA: 61.0%; purity: 98.6%) of 2-aminosulfonyl-N,N-dimethylnicotinamide.

Example 4

Procedures of Example 2 are repeated except that a step of dropping thereinto with agitation for about 20 minutes 12.5 g (0.125 mole) of concentrated sulfuric acid at a temperature of 5 to 20 °C after the completion of the oxidation reaction, is carried out between after the completion of the salt formation reaction and before the oxidation reaction to obtain 10.3 g (yield based on MPCA: 89.2%) of ammonium salt of N,N-dimethylnicotinamide-2-sulfinic acid.

Referential Example 4

The reaction product obtained according to the oxidation reaction in Example 4 is used as it is, and procedures of Referential Example 2 are repeated to obtain 6.82 g (yield based on MPCA: 59.6%; purity: 93.8%) of 2-aminosulfonyl-N,N-dimethylnicotinamide.

Example 5

Preparation of ACPS Salt of the Present Invention:

(Salt Formation Reaction)

A 300 mℓ four-necked flask equipped with thermometer, dropping funnel and stirrer is charged with 5.0 g (0.0275 mole) of 2-mercapto-N,N-dimethylnicotinamide, 1.2 g (0.03 mole) of sodium hydroxide and 15 mℓ of water, followed by dissolving with agitation and by reacting at room temperature for 10 minutes to obtain a reaction product containing sodium-N,N-dimethylnicotinamide-2-thiolate.

Separately, a reaction product obtained by carrying out the same reaction as above is thickened, and the deposited crystals are filtered off and dried to isolate 5.53 g of sodium-N,N-dimethylnicotinamide-2-thiolate having a melting point of 260 to 268 °C (a little decomposed).

(Oxidation Reaction)

Into the reaction product obtained according to the above salt formation reaction, as it is, is dropped with agitation for about 30 minutes 6.23 g (0.055 mole) of 30% aqueous hydrogen peroxide solution at a temperature of 10 to 20 °C externally cooling for reacting to obtain a reaction product containing sodium N,N-dimethylnicotinamide-2-sulfinate.

Separately, a reaction product obtained by carrying out the salt formation reaction and oxidation reaction in the same manner as above is thickened and dried to isolate 6.05 g (yield based on MPCA: 93.2%) of sodium N,N-dimethylnicotinamide-2-sulfinate having a melting point of 260 to 265 °C (colored brown, decomposed).

Referential Example 5

Preparation of APCA:

(Amination Reaction)

To the reaction product obtained according to the oxidation reaction in Example 5, as it is, is added 2.48 g (0.03 mole) of sodium acetate at a temperature of 10 to 30 °C, and 4.03 g (0.0357 mole) of hydroxylamine-o-sulfonic acid is then added for reacting with agitation for about 5 hours. Crystals deposited at about 20 °C is filtered, washed with water and dried to obtain 4.56 g (yield based on MPCA: 72.4%; purity: 95.3%) of 2-aminosulfonyl-N,N-dimethylnicotinamide.

Referential Example 6

Procedures of Referential Example 5 are repeated except that 1.83 g (0.03 mole) of 28% ammonia water in place of 2.48 g of sodium acetate is used and that the reaction time is 3 hours in place of 5 hours to obtain 4.66 g (yield based on MPCA: 74.0%; purity: 98.2%) of 2-aminosulfonyl-N,N-dimethylnicotinamide.

Referential Example 7

Procedures of Referential Example 5 are repeated except that 4.16 g (0.0412 mole) of triethylamine in place of 2.48 g of sodium acetate is used and that the reaction time is 3 hours in place of 5 hours to obtain 4.35 g (yield based on MPCA: 69.1%; puriy: 97.1%) of 2-aminosulfonyl-N,N-dimethylnicotinamide.

Example 6

Procedures of Example 5 are repeated except that 15 mℓ of water used in the salt formation reaction of Example 5 is replaced by 7.5 mℓ thereof to obtain 6.08 g (yield based on MPCA: 93.7%) of sodium N,N-dimethylnicotinamide-2-sulfinate.

Referential Exampled 8

Procedures of Referential Example 5 are repeated except that the reaction product obtained according to the oxidation reaction of Example 6 is used as it is, that a step of adding 2.48 g of sodium acetate followed by adding 4.03 g of hydroxylamine-o-sulfonic acid is replaced by a step of adding 4.03 g (0.0357 mole) of hydroxylamine-o-sulfonic acid followed by adding 1.83 g (0.03 mole) of 28% ammonia water, and that the reaction time is 3 hours in place of 5 hours to obtain 4.77 g (yield based on MPCA: 75.8%; purity: 98.3%) of 2-aminosulfonyl-N,N-dimethylnicotinamide.

Example 7

Procedures of Example 5 are repeated except that 1.8 g (0.03 mole) of 28% ammonia water in place of 1.2 g of sodium hydroxide is used to obtain 5.90 g (yield based on MPCA: 92.9%) of ammonium salt of N,N-dimethylnicotinamide-2-sulfinic acid.

Referential Example 9

Procedures of Referential Example 5 are repeated except that the reaction product obtained according to the oxidation reaction of Example 7 is used as it is, and that the reaction time is 6 hours in place of 5 hours to obtain 4.68 g (yield based on MPCA: 74.3%; purity: 98.0%) of 2-aminosulfonyl-N,N-dimethylnicotinamide.

Example 8

Preparation of ACPS Salt of the Present Invention:

(Polysulfidation Reaction)

A mixture of 55.4 g of 2-chloro-N,N-dimethylnicotinamide, 24 g of 70% purity sodium hydrosulfide, 9.6 g of sulfur, 12 g of sodium hydroxide and 15 mℓ of water is refluxed by heating with agitation for about 2 hours to obtain a reaction product containing 68.7 g of sodium salt of N,N-dimethylnicotinamide-2-(poly)-sulfide.

(Oxidation Reaction)

To the reaction product obtained according to the above polysulfidation reaction in Example 8 are added 180 mℓ of water and 18 g of a 40% aqueous sodium hydroxide solution, followed by dropping thereinto with agitation for 30 minutes 75.8 g (0.78 mole) of 35% aqueous hydrogen peroxide solution at a temperature of 10 to 20 °C for reacting to obtain a reaction product containing sodium N,N-dimethylnicotinamide-2-sulfinate.

The reaction product thus obtained is subjected to filtration to filter off free sulfur, and the sulfur is washed with water to obtain filtrate and washing liquor.

Separately, polysulfidation reaction and oxidation reaction are carried out in the same manner as above to obtain a reaction product. Similarly, free sulfur is removed from the reaction product, followed by thickening and drying to isolate 65.0 g (yield based on ACHP: 91.8%) of sodium N,N-dimethylnicotinamide-2-sulfinate having a melting point of 258 to 265 °C (colored brown, decomposed).

Referential Example 10

Preparation of APCA:

(Amination Reaction)

To a combined solution of the filtrate and the washing liquor obtained according to the oxidation reaction in Example 8 is added 54.24 g (0.48 mole) of hydroxylamine-o-sulfonic acid with agitation at 10 °C or lower to be dissolved, and 48 g (0.48 mole) of 40% aqueous sodium hydroxide solution is then dropped thereinto to be reacted with agitation for 3 hours at a temperature of 10 to 20 °C.

A reaction product thus obtained is filtered to obtain crystals, which are washed with water and dried to obtain 52.5 g (yield based on ACHP: 76.4%; purity: 96.5%) of 2-aminosulfonyl-N,N-dimethylnicotinamide.

**Claims**

**1.** An aminocarbonyl-substituted pyridinesulfinic acid having the general formula (I):

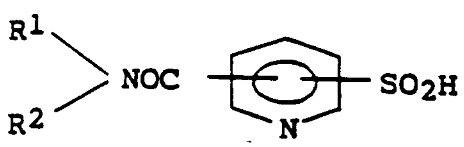

... (I)

where $R^1$ and $R^2$ are a hydrogen atom or a $C_1$-$C_6$ alkyl group respectively, or a salt thereof.

**2.** A compound as claimed in claim 1, having the general formula (I"):

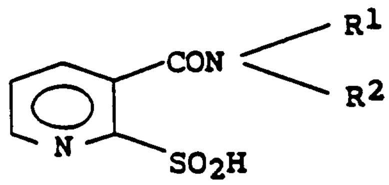

... (I")

where $R^1$ and $R^2$ are as defined in claim 1, or a salt thereof.

**3.** A compound as claimed in claim 1, having the general formula:

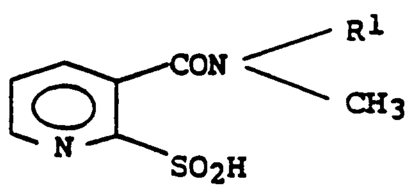

where $R^1$ is as defined in claim 1, or a salt thereof.

**4.** N,N-dimethylnicotinamide-2-sulfinic acid, or the sodium or ammonium salt thereof.

**5.** A process for preparing an aminocarbonyl-substituted pyridinesulfinic acid or a salt thereof, which process comprises (1) reacting a mercapto-substituted pyridinecarboxylic acid amide compound having the general formula (II):

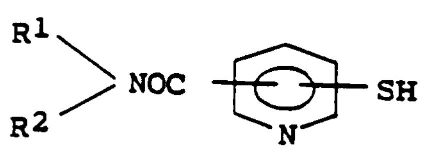

... (II)

where $R^1$ and $R^2$ are a hydrogen atom or a $C_1$-$C_6$ alkyl group respectively, with a salt-forming substance to form a salt of the carboxylic acid amide compound, and (2) reacting the salt of the carboxylic acid amide compound with hydrogen peroxide to prepare aminocarbonyl-substituted pyridine-sulfinic acid having the general formula (I):

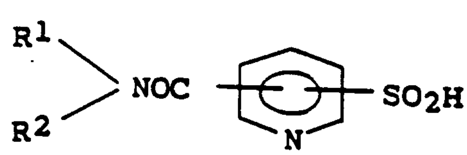

... (I)

where $R^1$ and $R^2$ are as defined above, or a salt thereof.

**6.** A process as claimed in claim 5, characterized in that the mercapto-substituted pyridinecarboxylic acid amide compound is one having the general formula (II'):

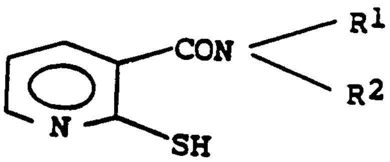

... (II')

where $R^1$ and $R^2$ are as defined in claim 5, and the aminocarbonyl-substituted pyridinesulfinic acid or salt thereof is one having the general formula (I''):

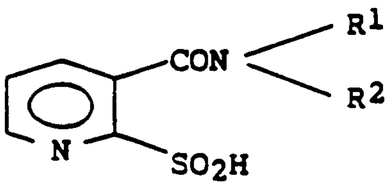

... (I")

where $R^1$ and $R^2$ are as defined in claim 5, or a salt thereof.

**7.** A process as claimed in claim 5, characterized in that the reaction temperature of the salt formation reaction is 0 to 50 °C, and the reaction temperature of the oxidation reaction is 0 to 100 °C.

**8.** A process as claimed in claim 5, characterized in that the salt-forming substance is sodium hydroxide or ammonia.

**9.** A process for preparing an aminocarbonyl-substituted pyridinesulfinic acid or an alkali metal salt thereof, which process comprises (1) reacting an aminocarbonyl-substituted halogenopyridine compound having the general formula (III):

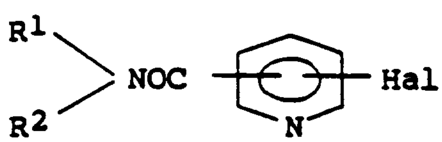

... (III)

where $R^1$ and $R^2$ are a hydrogen atom or a $C_1$-$C_6$ alkyl group respectively, and Hal is a halogen atom, with a polysulfide having the formula: $M'_2S_x$ where M' is an alkali metal element and x is 2 to 8, to obtain an alkali metal salt of a pyridinecarboxylic acid amide (poly)sulfide, and (2) reacting the alkali metal salt of the (poly)sulfide with hydrogen peroxide to prepare an aminocarbonyl-substituted pyridinesulfinic acid or alkali metal salt thereof having the general formula (I'):

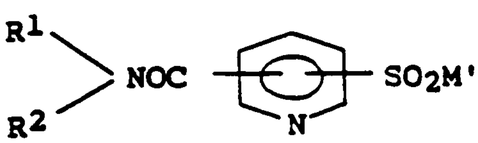

... (I')

where $R^1$, $R^2$ and M' are as defined above.

**10.** A process as claimed in claim 9, characterized in that the aminocarbonyl-substituted halogenopyridine compound is one having the general formula (III'):

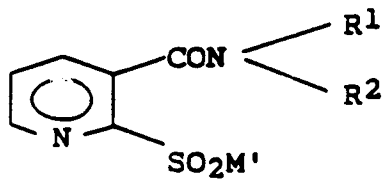

... (III')

where $R^1$, $R^2$ and M' are as defined in claim 9.

**11.** A process as claimed in claim 9, characterized in that the reaction temperature of the reaction with the polysulfide is 0 ° C to the reflux temperature, and the reaction temperature of the oxidation reaction is 0 to 100 ° C.

**12.** A process as claimed in claim 9, characterized in that M' constituting the polysulfide is a sodium atom.

**13.** A process of using N,N-dimethylnicotinamide-2-sulfinic acid or a salt thereof, as an intermediate for the manufacture of N-[(4,6-dimethoxypyrimidine-2-yl)aminocarbonyl]-3-dimethylaminocarbonyl-2-pyridinesulfonamide as a herbicide.

**Patentansprüche**

**1.** Aminocarbonyl-substituierte Pyridinsulfinsäure mit der allgemeinen Formel (I):

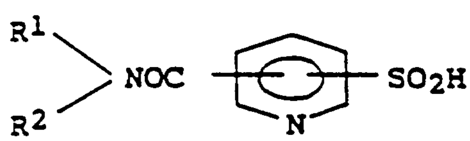

... (I)

worin $R^1$ und $R^2$ jeweils ein Wasserstoffatom oder eine $C_{1-6}$-Alkylgruppe sind, oder Salz davon.

**2.** Verbindung gemäß Anspruch 1 mit der allgemeinen Formel (I"):

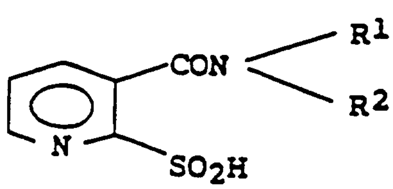

... (I")

worin $R^1$ und $R^2$ wie in Anspruch 1 definiert sind, oder Salz davon.

**3.** Verbindung gemäß Anspruch 1 mit der allgemeinen Formel:

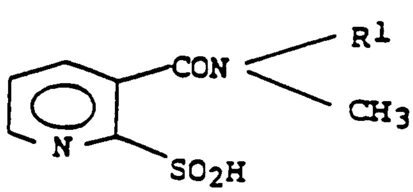

worin $R^1$ wie in Anspruch 1 definiert ist, oder Salz davon.

**4.** N,N-Dimethylnicotinamid-2-sulfinsäure oder das Natrium- oder Ammoniumsalz davon.

**5.** Verfahren zur Herstellung einer Aminocarbonyl-substituierten Pyridinsulfinsäure oder eines Salzes davon, wobei man (1) eine Mercapto-substituierte Pyridincarboxylsäureamid-Verbindung mit der allgemeinen Formel (II):

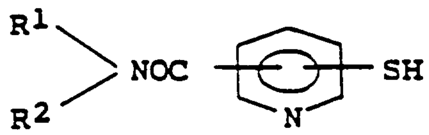

... (II)

worin $R^1$ und $R^2$ jeweils ein Wasserstoffatom oder eine $C_{1-6}$-Alkylgrupe sind, mit einer Salzbildungssubstanz umsetzt, um ein Salz der Carboxylsäureamid-Verbindung zu bilden, und man (2) das Salz der Carboxylsäureamid-Verbindung mit Wasserstoffperoxid reagieren läßt, um die Aminocarbonyl-substituierte Pyridinsulfinsäure der allgemeinen Formel (I):

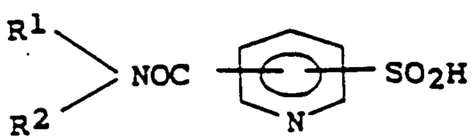

... (I)

worin $R^1$ und $R^2$ wie oben definiert sind, oder ein Salz davon herzustellen.

**6.** Verfahren gemäß Anspruch 5,
dadurch **gekennzeichnet**, daß
die Mercapto-substituierte Pyridincarboxylsäureamid-Verbindung die allgemeine Formel (II') aufweist:

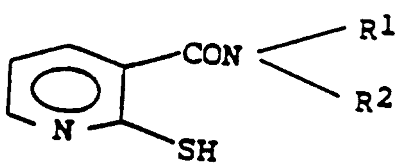

... (II')

worin $R^1$ und $R^2$ wie in Anspruch 5 definiert sind, und daß die Aminocarbonyl-substituierte Pyridinsulfinsäure oder das Salz davon die allgemeine Formel (I") aufweisen:

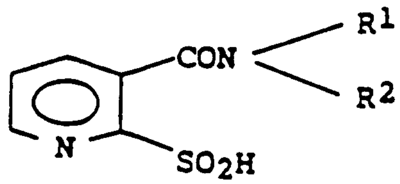

... (I")

worin $R^1$ und $R^2$ wie in Anspruch 5 definiert sind, und gegebenenfalls ein Salz davon vorliegt.

7. Verfahren gemäß Anspruch 5, dadurch **gekennzeichnet**, daß die Reaktionstemperatur der Salzbildungsreaktion 0 bis 50 °C und die Reaktionstemperatur der Oxidationsreaktion 0 bis 100 °C betragen.

8. Verfahren gemäß Anspruch 5, dadurch **gekennzeichnet**, daß die Salzbildungssubstanz Natriumhydroxid oder Ammoniak ist.

9. Verfahren zur Herstellung einer Aminocarbonyl-substituierten Pyridinsulfinsäure oder eines Alkalimetallsalzes davon, wobei man (1) eine Aminocarbonyl-substituierte Halogenpyridin-Verbindung der allgemeinen Formel (III):

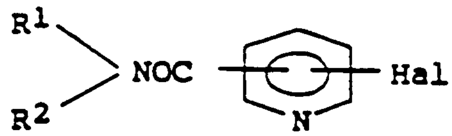

... (III)

worin $R^1$ und $R^2$ jeweils ein Wasserstoffatom oder eine $C_{1-6}$-Alkylgruppe und Hal ein Halogenatom sind, mit einem Polysulfid der Formel: $M'_2S_x$, worin M' ein Alkalimetall und x 2 bis 8 sind, reagieren läßt, um ein Alkalimetallsalz eines Pyridincarboxylsäureamid(poly)sulfids zu erhalten, und man (2) das Alkalimetallsalz des (Poly)sulfids mit Wasserstoffperoxid zur Reaktion bringt, um eine Aminocarbonyl-substituierte Pyridinsulfinsäure oder ein Alkalimetallsalz davon der allgemeinen Formel (I') herzustellen:

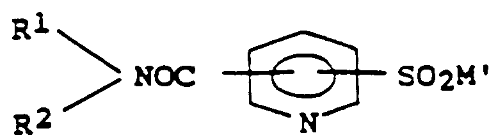

... (I')

worin $R^1$, $R^2$ und M' wie oben definiert sind.

10. Verfahren gemäß Anspruch 9, dadurch **gekennzeichnet**, daß die Aminocarbonyl-substituierte Pyridin-Verbindung die allgemeine Formel (III') aufweist:

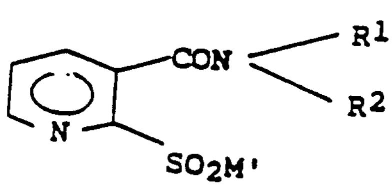

... (III')

worin $R^1$, $R^2$ und M' wie in Anspruch 9 definiert sind.

11. Verfahren gemäß Anspruch 9, dadurch **gekennzeichnet**, daß die Reaktionstemperatur der Reaktion mit dem Polysulfid 0 °C bis Rückflußtemperatur und die Reaktionstemperatur der Oxidationsreaktion 0 bis 100 °C betragen.

12. Verfahren gemäß Anspruch 9, dadurch **gekennzeichnet**, daß

M' im Polysulfid ein Natriumatom ist.

**13.** Verfahren zur Verwendung von N,N-Dimethylnicotinamid-2-sulfinsäure oder eines Salzes davon als Zwischenprodukt zur Herstellung von N-((4,6-Dimethoxypyrimidin-2-yl)aminocarbonyl)-3-dimethylaminocarbonyl-2-pyridinsulfonamid als Herbizid.

**Revendications**

**1.** Acide pyridinesulfinique aminocarbonyle-substitué ayant la formule générale (I) :

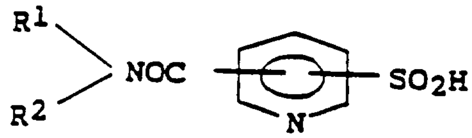

... (I)

où $R^1$ et $R^2$ représentent un atome d'hydrogène ou un groupe alkyle en $C_1$-$C_6$ respectivement, ou un de ses sels.

**2.** Composé selon la revendication 1 qui a la formule générale (I") :

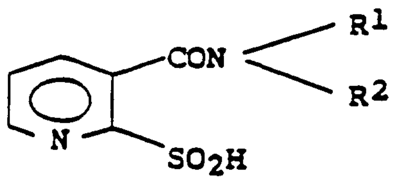

... (I")

où $R^1$ et $R^2$ sont tels que définis à la revendication 1, ou un de ses sels.

**3.** Composé selon la revendication 1 qui a la formule générale :

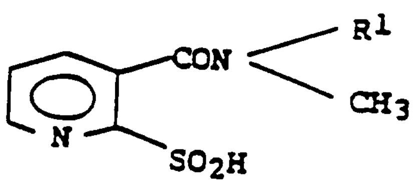

où $R^1$ est tel que défini à la revendication 1 ou un de ses sels.

**4.** Acide N,N-diméthylnicotinamide-2-sulfinique ou son sel de sodium ou d'ammonium.

**5.** Procédé de préparation d'un acide pyridinesulfinique aminocarbonyle-substitué ou un de ses sels, ce procédé comprend (1) de faire réagir un composé amide d'acide pyridinecarboxylique mercapto-substitué ayant la formule générale (II) :

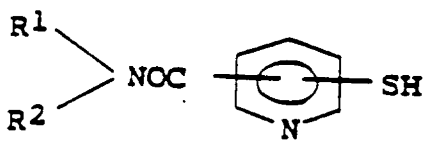

... (II)

où $R^1$ et $R^2$ représentent un atome d'hydrogène ou un groupe alkyle en $C_1$-$C_6$ respectivement, avec

une substnce formant un sel pour former un sel du composé amide d'acide carboxylique, et (2) de faire réagir le sel du composé amide d'acide carboxylique avec du peroxyde d'hydrogène pour préparer l'acide pyridinesulfinique aminocarbonyle-substitué ayant la formule générale (I) :

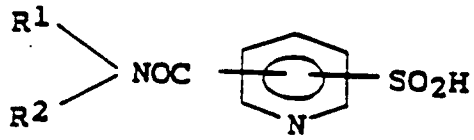

... (I)

où $R^1$ et $R^2$ sont tels que définis ci-dessus, ou un de ses sels.

**6.** Procédé selon la revendication 5, caractérisé en ce que le composé amide d'acide pyridinecarboxylique mercapto-substitué est l'un des composés ayant la formule générale (II') :

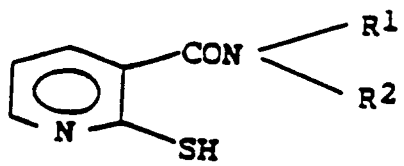

... (II')

où $R^1$ et $R^2$ sont tels que définis à la revendication 5, et l'acide pyridinesulfinique aminocarbonyle-substitué ou un de ses sels est l'un des composés ayant la formule générale (I") :

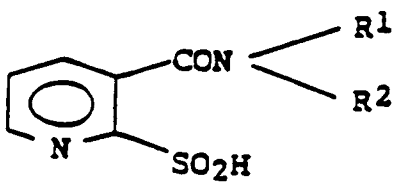

... (I")

où $R^1$ et $R^2$ sont tels que définis à la revendication 5, ou un de ses sels.

**7.** Procédé selon la revendication 5, caractérisé en ce que la température de réaction de la réaction de formation du sel est comprise entre 0 et 50 °C, et la température de réaction d'oxydation est comprise entre 0 et 100 °C.

**8.** Procédé selon la revendication 5, caractérisé en ce que la substance formant le sel est l'hydroxyde de sodium ou l'ammoniac.

**9.** Procédé de préparation d'un acide pyridinesulfinique aminocarbonyle-substitué ou un de ses sels de métal alcalin, ce procédé comprend (1) de faire réagir un composé halogénopyridine aminocarbonyle-substitué ayant la formule générale (III) :

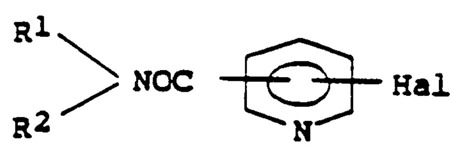

... (III)

où $R^1$ et $R^2$ représentent un atome d'hydrogène ou un groupe alkyle en $C_1$-$C_6$ respectivement, et Hal est un atome d'halogène, avec un polysulfure ayant la formule : $M'_2S_x$ où M' est un élément de métal alcalin et x représente 2 à 8, pour donner un sel de métal alcalin d'un polysulfure d'amide d'acide

pyridinecarboxylique et (2) de faire réagir le sel de métal alcalin du polysulfure avec du peroxyde d'hydrogène pour préparer un acide pyridinesulfinique aminocarbonyle-substitué ou un de ses sels de métal alcalin ayant la formule générale (I') :

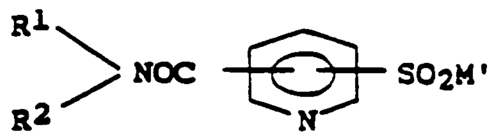

... (I')

où $R^1$, $R^2$ et M' sont tels que définis ci-dessus.

**10.** Procédé selon la revendication 9, caractérisé en ce que le composé halogénopyridine aminocarbonyle-substitué est un composé ayant la formule générale (III') :

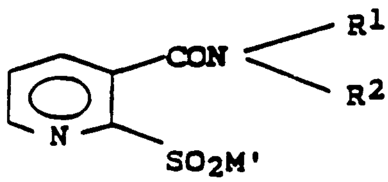

... (III')

où $R^1$, $R^2$ et M' sont tels que définis à la revendication 9.

**11.** Procédé selon la revendication 9, caractérisé en ce que la température de réaction de la réaction avec le polysulfure est comprise entre 0 ° C et la température de reflux, et la température de réaction de la réaction d'oxydation est comprise entre 0 et 100 ° C.

**12.** Procédé selon la revendication 9, caractérisé en ce que M' constituant le polysulfure est un atome de sodium.

**13.** Procédé utilisant l'acide N,N-diméthylnicotinamide-2-sulfinique ou un de ses sels comme intermédiaire de la préparation du N-[(4,6-diméthoxypyrimidine-2-yl)aminocarbonyl]-3-diméthylaminocarbonyl-2-pyridinesulfonamide en tant qu'herbicide.